# FASCICULE DE BREVET EUROPEEN

(11) **EP 0 576 337 B1**
(45) Date de publication et mention de la délivrance du brevet: **05.03.1997**
(21) Numéro de dépôt: 93401572.8
(22) Date de dépôt: 18.06.1993
(51) Int. Cl.: A61F 2/08, A61B 17/06, A61B 17/04

(54) **Matériel utilisable pour la réparation du tendon d'achille rompu**
Vorrichtung zur Reparatur der Achillessehne
Device for the repair of a ruptured achilles tendon

(30) Priorité: 25.06.1992 FR 9208059
(43) Date de publication de la demande: 29.12.1993
(73) Titulaire: SOCIETE B.F.L MEDICAL SA, 63306 Thiers Cedex (FR)
(72) Inventeur: Delponte, Patrick, F-29215 Guipavas (FR)
(74) Mandataire: Ravina, Bernard

(56) Documents cités:
- EP-A- 0 478 949
- EP-A- 0 513 736
- DE-A- 3 227 984
- FR-A- 494 960
- FR-A- 2 653 658
- US-A- 3 265 070
- US-A- 4 705 040

## Description

La présente invention a pour objet un matériel utilisable pour la réparation du tendon d'Achille rompu.

Pour la réparation du tendon d'Achille, le chirurgien, conformément à une méthode déjà connue, effectue d'abord une incision au dessus du talon de façon à dégager le tendon. Ensuite, il insère dans le tendon rompu, axialement, plusieurs fils métalliques destinés à lier l'un à l'autre les deux fragments.

Il procède à la mise en place d'un harpon sur chaque fil métallique, les harpons étant destinés à venir s'ancrer dans le fragment supérieur à deux centimètres environ de la zone de rupture.

Après cette opération, il insère sur l'autre extrémité du fil une rondelle puis un organe d'arrêt.

Les fils étant mis en tension et les deux fragments de ligaments étant télescopés, les rondelles sont amenées en pression contre le tronçon inférieur du ligament et maintenues en position par les organes d'arrêt.

Ces derniers se présentent, par exemple, sous la forme d'une perle en matériau ductile pour pouvoir être déformée sur le fil. Ensuite, il referme la plaie.

Après réparation complète du ligament, les fils seront retirés.

On conçoit que cette technique bien qu'efficace sur le plan du résultat présente quelques inconvénients, tels que notamment hospitalisation de durée relativement importante, rééducation retardée, etc.

On connaît du document EP-478 949, un dispositif d'aide à l'implantation d'une suture utilisable pour maintenir l'une contre l'autre en vue de cicatrisation les berges d'un tendon rompu. Ce dispositif comprend une canule pourvue en extrémité d'une pointe détachable à laquelle est fixé un fil de suture engagé dans la canule. La pointe détachable a pour but d'ouvrir le passage à la canule dans le tissu anatomique.

La présente invention a pour objet de résoudre les inconvénients précédemment énoncés en mettant en oeuvre un matériel de réparation du tendon, par l'emploi duquel est réduite l'importance de l'agression, diminuée la durée de l'hospitalisation, avancée la période de rééducation.

Un autre but de la présente invention est la mise en oeuvre d'un matériel permettant une intervention rapide et simple à la portée de tous les opérateurs.

A cet effet, le matériel pour la réparation chirurgicale d'un tendon rompu, par exemple, le tendon d'Achille, comprenant un lien, un harpon, une rondelle d'appui et un organe d'arrêt déformable, ladite rondelle et ledit organe d'arrêt étant destinés à être engagés sur le lien, d'abord la rondelle ensuite l'organe d'arrêt et ledit organe d'arrêt étant destiné à être bloqué sur le lien par déformation, est caractérisé en ce que :
- il comprend une aiguille en extrémité de laquelle le lien est fixé par sertissage,
- le lien est souple et présente une résistance à la traction appropriée,
- le lien souple est équipé en extrémité libre d'un embout de préhension fixé par sertissage,
- l'harpon est fixé sur le lien souple à distance de l'aiguille,
- ladite rondelle et ledit organe d'arrêt sont destinés à être engagés sur ledit lien du côté de l'aiguille.

D'autres avantages et caractéristiques apparaîtront à la lecture de la description d'une forme préférée de réalisation donnée à titre non limitatif en se référant aux dessins annexés en lesquels :

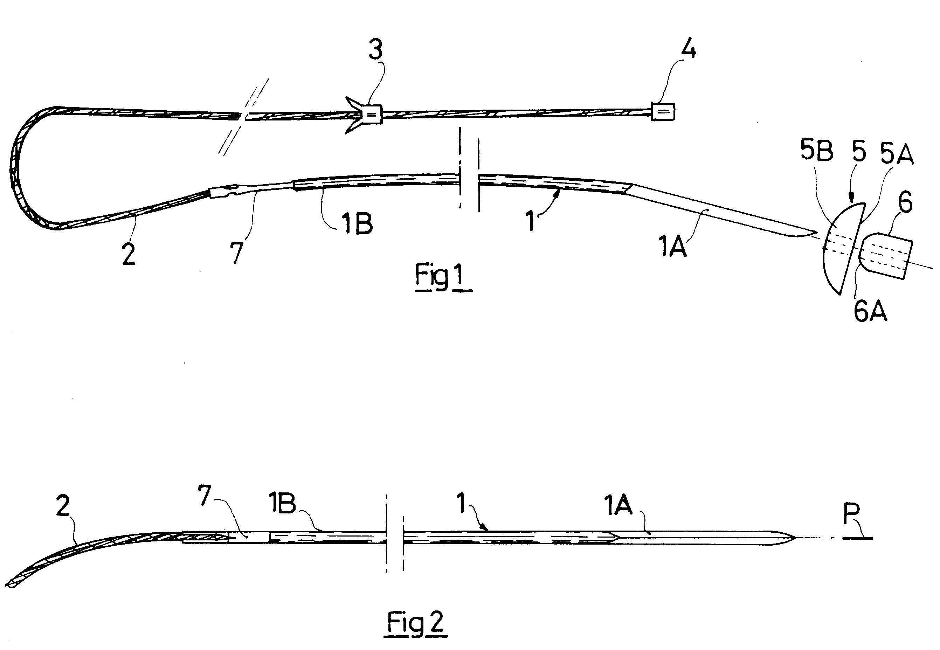

- la figure 1 est une vue d'ensemble du matériel,
- la figure 2 est une vue de dessus de l'aiguille,

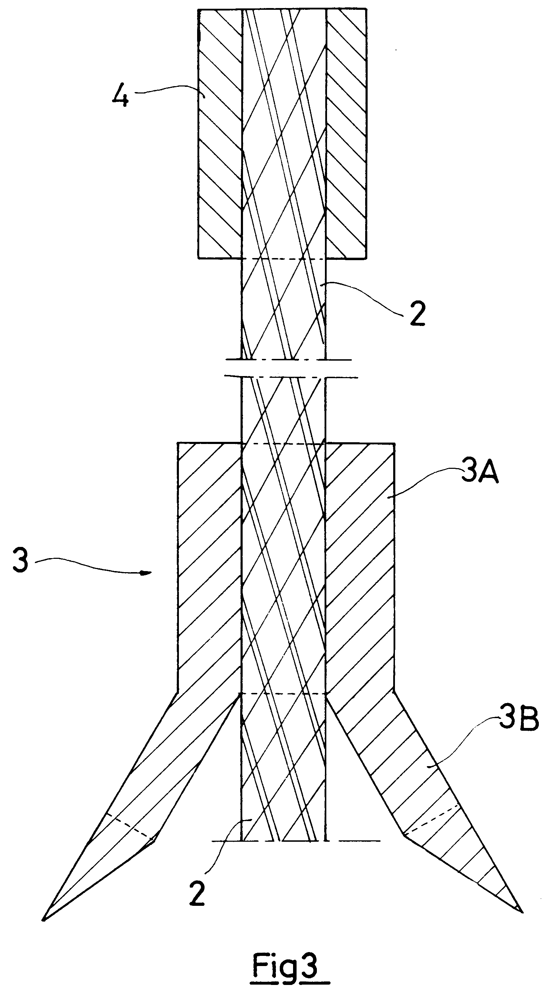

- la figure 3 est une vue en coupe suivant une échelle agrandie d'un harpon et d'un embout,
- les figures 4 à 9 illustrent les étapes de pose du lien souple.

Tel que représenté, le matériel pour la réparation chirurgicale d'un tendon rompu, par exemple un tendon d'Achille, est constitué par une aiguille 1 en extrémité de laquelle est fixé par sertissage un lien souple 2, présentant une résistance à la traction appropriée, équipé à distance de l'aiguille d'un harpon 3 et en extrémité libre, d'un embout 4 de préhension, fixé par sertissage, le dit matériel comprenant en outre une rondelle d'appui 5 et un organe d'arrêt 6 déformable, destinés tous deux à être engagés sur le lien.

Selon la forme préférée de réalisation, l'aiguille 1 présente une partie avant 1A de section droite triangulaire suivie d'une partie arrière 1B de section droite circulaire.

La partie avant 1A et la partie 1B de l'aiguille sont de préférence rectilignes ou sensiblement rectilignes.

Préférentiellement, elles forment entre elles un angle obtu.

Selon une forme préférée de réalisation, la section droite de la partie avant forme un triangle équilatéral et la dite section est orientée en sorte que l'une des hauteurs du triangle soit contenue dans le plan contenant les axes longitudinaux des parties avant 1A et arrière 1B de l'aiguille.

A titre d'exemple purement indicatif, la partie avant présente une longueur de trois centimètres tandis que la partie arrière présente une longueur de neuf à dix centimètres.

Toujours à titre d'exemple, la valeur de l'angle sera sensiblement égale à 170 degrés.

L'aiguille, réalisée en matériau approprié tel que de l'acier inoxydable, peut être modelée à volonté pour présenter, par exemple, une courbure adaptée au type de rupture traité.

Toujours selon la forme préférée de réalisation, il est prévu un repère, pratiqué en extrémité arrière de la partie arrière de l'aiguille.

Ce repère est constitué par deux méplats 7 diamétralement opposés.

Préférentiellement, ces deux méplats sont perpendiculaires au plan géométrique (P) contenant l'axe longitudinal de la partie 1B de l'aiguille et l'axe longitudinal de la partie avant 1A de cette dernière.

Selon la forme préférée de réalisation, le lien souple 2 est constitué par une tresse formée à partir de faisceaux comportant chacun une pluralité de filaments et par une âme centrale autour de laquelle est formée la tresse, la dite âme étant formée par un faisceau de filaments.

L'harpon 3 est formé d'un corps tubulaire 3A engagé sur le lien souple, prolongé en direction de l'aiguille par deux pointes 3B opposées et divergentes, le corps tubulaire 3A étant déformé sur le lien souple 2 de façon à être bloqué en position sur ce dernier.

A titre purement indicatif, la longueur du lien mesurée entre l'harpon 3 et l'extrémité arrière de l'aiguille est égale à au moins une fois la longueur de l'aiguille.

Préférentiellement, la longueur du lien entre l'harpon 3 et l'extrémité arrière de l'aiguille est sensiblement égale à deux fois la longueur de l'aiguille.

L'embout 4 est un élément tubulaire recevant l'extrémité libre du lien et déformé sur la dite extrémité de façon à être fixé sur le dit lien.

Il y a lieu de noter que le lien souple présente une longueur de quelques centimètres entre l'harpon et l'embout.

La rondelle d'appui 5 présente un orifice d'engagement de l'aiguille puis du lien. Une des grandes faces de la rondelle est plane 5A tandis que l'autre est convexe 5B, ces deux faces étant perpendiculaires à l'axe de l'orifice.

L'organe d'arrêt 6 est constitué par un élément cylindrique perforé suivant son axe pour le passage de l'aiguille et du lien. Cet organe présente en extrémité une face convexe 6A.

Cet organe d'arrêt 6 est réalisé par un matériau ductile pour pouvoir être écrasé sur le lien.

En règle générale deux matériels tels que précédemment décrits suffisent pour la réparation d'un tendon d'achille rompu.

On va maintenant exposer l'utilisation du matériel.

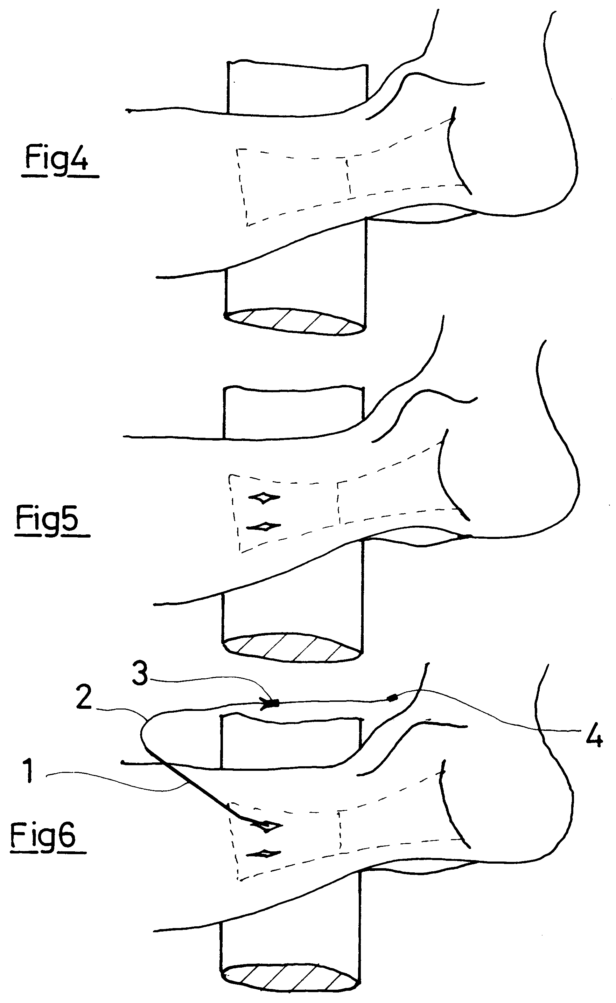

Le pied étant maintenu en équinisme sur un appui placé sous le coup de pied, le chirurgien repère au palper et balise au crayon dermographique les limites du tendon et de la zone de rupture (figure 4).

Deux mouchetures cutanées légèrement latéralisées sont pratiquées à quatre centimètres de la zone de rupture (figure 5).

Par ces mouchetures sont introduites dans le tendon, les aiguilles et leur lien souple (figure 6).

L'introduction de l'aiguille s'effectue suivant l'axe du tendon et la progression et la position de l'aiguille sont contrôlées au palper.

La position de l'aiguille autour de son axe peut être facilement déterminée par observation de la position des méplats, par lesquels l'aiguille peut être manoeuvrée à l'aide d'une pince.

Il faut noter que lors de la progression de l'aiguille dans le tendon, le plan géométrique P est sensiblement perpendiculaire au plan du tendon.

Il est très facile avec une telle aiguille de franchir la zone de rupture et de traverser axialement le moignon distal.

Lorsque la pointe de l'aiguille parvient au niveau de l'insertion calcanééne, le chirurgien l'incline latéralement de façon à ce qu'elle puisse émerger dans la fossette rétro-malléolaire interne ou externe selon le cas.

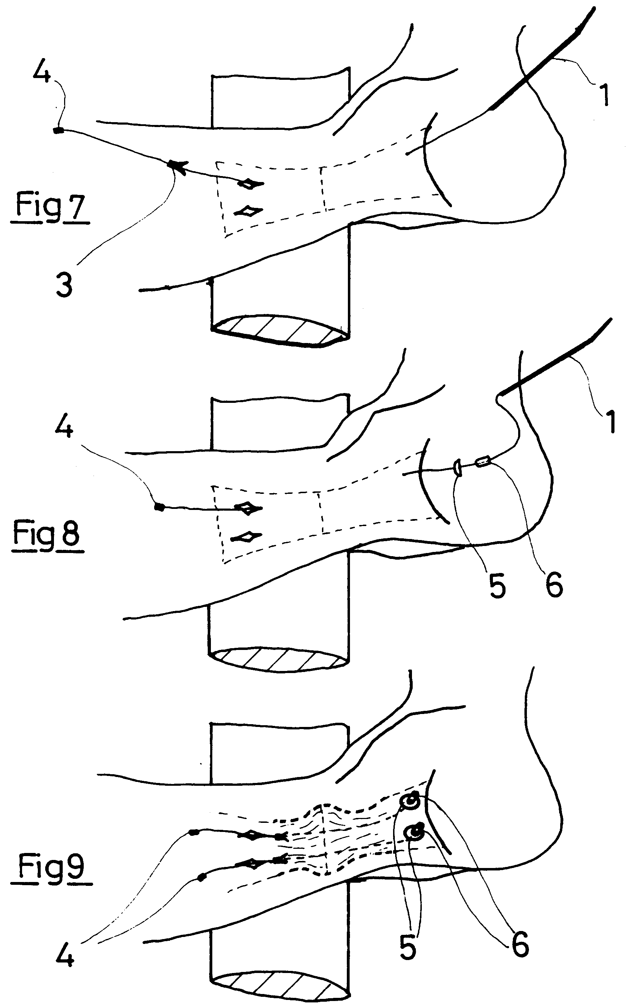

L'aiguille étant extraite de la fossette et tirée, le lien souple s'engage dans le ligament rompu et le harpon vient s'ancrer dans la gaine du moignon supérieur (figures 7 et 8).

Après introduction des deux liens dans le tendon comme il vient d'être décrit, le chirurgien engage sur chaque aiguille puis sur le lien d'abord la rondelle d'appui 5 et puis l'organe d'arrêt 6 (figure 8).

La rondelle 5 est engagée en sorte que sa face convexe 5B soit tournée vers le pied du malade et l'organe d'arrêt est engagé de façon que sa face convexe 6A soit tournée vers la face plane 5A de la rondelle 5.

Le pied étant maintenu en équinisme le chirurgien procède à la mise en tension des liens. Lors de cette mise en tension, la face convexe de chaque rondelle prend appui contre la facette malléolaire.

Cette mise en tension des liens a pour but d'assurer un télescopage des fibres des deux moignons du tendon rompu à l'intérieur de la gaine.

Le télescopage est décelable au palper sous la forme d'un léger bourrelet au niveau de la zone préalablement déhiscente (en pointillés sur la figure 9).

Pour conserver cette mise en tension, les organes d'arrêt sont amenés au contact de leur rondelle respective et sont déformés sur le lien souple.

Le lien souple est ensuite cisaillé au ras de l'organe d'arrêt (figure 9).

Il faut noter que la portion du lien souple comprise entre l'harpon et l'embout et se trouvant en dehors de la jambe du malade n'est pas cisaillée et demeure en place comme on peut le voir en figure 9.

Le lien souple demeure dans le tendon quatre à cinq semaines et ensuite le chirurgien procède à son ablation par traction sur l'embout 6 après avoir procédé au retrait de l'organe d'arrêt.
Ce retrait s'effectue facilement en cisaillant le lien souple entre la rondelle 5 et l'organe d'arrêt 6.

## Revendications

1. Matériel pour la réparation chirurgicale d'un tendon rompu, par exemple, le tendon d'Achille, comprenant un lien (2), un harpon (3), une rondelle d'appui (5) et un organe d'arrêt (6) déformable, ladite rondelle (5) et ledit organe d'arrêt (6) étant destinés à être engagés sur le lien (3), d'abord la rondelle (5) ensuite l'organe d'arrêt (6) et le dit organe d'arrêt (6) étant destiné à être bloqué sur le lien (3) par déformation, caractérisé en ce que :
- il comprend une aiguille (1) en extrémité de laquelle le lien (2) est fixé par sertissage,
- le lien (2) est souple et présente une résistance à la traction appropriée,
- le lien souple (2) est équipé en extremité libre d'un embout (4) de préhension fixé par sertissage,
- l'harpon (3) est fixé sur le lien souple (2) à distance de l'aiguille (1),
- ladite rondelle (5) et ledit organe d'arrêt sont destinés à être engagés sur ledit lien (3) du côté de l'aiguille (1).

2. Matériel selon la revendication 1 caractérisé en ce que l'aiguille (1) présente une partie avant (1A) de section droite triangulaire suivie d'une partie arrière (1B) de section droite circulaire.

3. Matériel selon la revendication 2 caractérisé en ce que la partie avant (1A) et la partie arrière (1B) forment entre elles un angle obtus.

4. Matériel selon la revendication 3 caractérise par un repère pratiqué en extrémité arrière de la partie arrière de l'aiguille.

5. Matériel selon la revendication 4 caractérisé en ce que le repère est constitué par deux méplats (7) diamétralement opposés.

6. Matériel selon la revendication 5 caractérisé en ce que les méplats (7) sont perpendiculaires au plan géométrique (P) contenant l'axe longitudinal de la partie arrière (1B) de l'aiguille et l'axe longitudinal de la partie avant (1A) de cette dernière.

7. Matériel selon la revendication 1 caractérisé en ce que le lien souple (2) est constitué par une tresse formée à partir de faisceaux comportant chacun une pluralité de filaments et par une âme centrale autour de laquelle est formée la tresse, la dite âme étant formée par un faisceau de filaments.

8. Matériel selon la revendication 1 caractérisé en ce que l'harpon (3) est formé d'un corps tubulaire (3A) engagé sur le lien souple, prolongé en direction de l'aiguille par deux pointes (3B) opposées et divergentes, le corps tubulaire (3A) étant déformé sur le lien souple (2) de façon à être bloqué en position sur ce dernier.

9. Matériel selon la revendication 1 caractérisé en ce que la longueur du lien (2), mesurée entre l'harpon (3) et l'extrémité arrière de l'aiguille, est égale à au moins une fois la longueur de l'aiguille.

10. Matériel selon la revendication 9 caractérisé en ce que la longueur du lien (2) entre l'harpon (3) et l'extrémité arrière de l'aiguille est sensiblement égale à deux fois la longueur de l'aiguille.

11. Matériel selon la revendication 1 caractérisé en ce que la rondelle (5), perpendiculairement à l'axe de son orifice, présente une première face plane (5A) et une seconde face (5B), convexe, opposée à la précédente et destinée à venir en pression contre la fossette rétro-malléoaire.

12. Matériel selon la revendication 1 caractérisé en ce que l'organe d'arrêt (6) est constitué par un élément cylindrique perforé axialement et présentant en extrémité une face convexe (6A) destinée à venir en appui contre la rondelle (5) le dit organe étant en un matériau ductile pour pouvoir être déformé sur le lien.

## Claims

1. Equipment for the surgical repair of a ruptured tendon, for example, the Achilles' tendon, comprising a tie (2), a harpoon (3), a support disk (5) and a deformable stop element (6), said disk (5) and said stop element (6) being intended to be engaged over the tie (3), first the disk (5) and then the stop element (6), and said stop element (6) being intended to be locked on to the tie (3) by deformation, characterised in that:
- it comprises a needle (1) to the end of which the tie (2) is fastened on by crimping,
- the tie (2) is flexible and has appropriate resistance to traction,
- the flexible tie (2) is fitted at its free end with a gripping terminal (4) fastened on by crimping,
- the harpoon (3) is fastened to the flexible tie (2) some distance away from the needle (1),
- said disk (5) and said stop element are intended to be engaged over said tie (3) on the side of the needle (1).

2. Equipment according to Claim 1 characterised in that the needle (1) has a front portion (1A) with a triangular cross section followed by a rear portion (1B) of circular cross section.

3. Equipment according to Claim 2 characterised in that the front portion (1A) and the rear portion (1B) form an obtuse angle between them.

4. Equipment according to Claim 3 characterised by a marker made at the rear end of the rear portion of the needle.

5. Equipment according to Claim 4 characterised in that the marker is constituted by two diametrically opposite flat surfaces.

6. Equipment according to Claim 5 characterised in that the flat surfaces (7) are perpendicular to the geometric plane (P) containing the longitudinal axis of the rear portion (1B) of the needle and the longitudinal axis of the front portion (1A) of the latter.

7. Equipment according to Claim 1 characterised in that the flexible tie (2) is constituted by a braid formed from clusters each comprising a plurality of filaments and by a central core around which the braid is formed, said core being formed by a bundle of filaments.

8. Equipment according to Claim 1 characterised in that the harpoon (3) is formed of a tubular body (3A) engaged over the flexible tie, extended in the direction of the needle by two opposite and divergent tips (3B), the tubular body (3A) being deformed over the flexible tie (2) so as to be locked in position over the latter.

9. Equipment according to Claim 1 characterised in that the length of the tie (2), measured between the harpoon (3) and the rear end of the needle, is equal to at least once the length of the needle.

10. Equipment according to Claim 9 characterised in that the length of the tie (2) between the harpoon (3) and the rear end of the needle is substantially equal to twice the length of the needle.

11. Equipment according to Claim 1 characterised in that the disk (5), perpendicular to the axis of its orifice, has a first plane face (5A) and a second face (5B) which is convex and opposite the preceding one and is intended to press against the retromalleolar fossa.

12. Equipment according to Claim 1 characterised in that the stop element (6) is constituted by a cylindrical component perforated axially and having at its end a convex face (6A) intended to bear against the disk (5), said element being made of a ductile material so as to be capable of being deformed over the tie.

## Patentansprüche

1. Material zur chirurgischen Reparatur einer gerissenen Sehne wie zum Beispiel der Achillessehne, das ein Verbindungsmittel (2), einen Anker (3), eine Stützscheibe (5) und ein deformierbares Halteorgan (6) aufweist, wobei die Scheibe (5) und das Halteorgan (6) dafür bestimmt sind, mit dem Verbindungsmittel (2) verbunden zu werden, und zwar zunächst die Scheibe (5) und dann das Halteorgan (6), und wobei das Halteorgan (6) dafür bestimmt ist, daß es durch Deformation auf dem Verbindungsglied (3) blockiert wird, gekennzeichnet durch die folgenden Merkmale:
- das Material weist eine Nadel (1) auf, an deren Ende das Verbindungsglied (2) durch eine Quetschverbindung fixiert ist,
- das Verbindungsglied (2) ist flexibel und weist eine angemessene Zugfestigkeit auf,
- das flexible Verbindungsglied (2) ist an einem freien Ende mit einem Greifnippel (4) versehen, der durch eine Quetschverbindung fixiert ist,
- der Anker (3) ist in einem Abstand von der Nadel (1) auf dem flexiblen Verbindungsglied (2) fixiert,
- die Scheibe (5) und das Halteorgan sind dafür bestimmt, daß sie mit dem Verbindungsglied (3) auf der Seite der Nadel (1) verbunden werden.

2. Material gemäß Anspruch 1, dadurch gekennzeichnet, daß die Nadel (1) einen vorderen Teil (1A) mit geradem dreieckigen Querschnitt aufweist, auf den ein hinterer Teil (1B) mit geradem kreisförmigen Querschnitt folgt.

3. Material gemäß Anspruch 2, dadurch gekennzeichnet, daß der vordere Teil (1A) und der hintere Teil (1B) zwischen sich einen stumpfen Winkel bilden.

4. Material gemäß Anspruch 3, dadurch gekennzeichnet, daß eine Markierung am hinteren Ende des hinteren Teils der Nadel ausgebildet ist.

5. Material gemäß Anspruch 4, dadurch gekennzeichnet, daß die Markierung durch zwei diametral gegenüberliegende Abflachungen gebildet ist.

6. Material gemäß Anspruch 5, dadurch gekennzeichnet, daß die Abflachungen (7) senkrecht zu der geometrischen Ebene (P) stehen, die die Längsachse des hinteren Teils (1B) der Nadel und die Längsachse des vorderen Teils (1A) dieser letzteren enthält.

7. Material gemäß Anspruch 1, dadurch gekennzeichnet, daß das flexible Verbindungsglied (2) aus einer Litze besteht, die aus Bündeln, von denen jedes eine Vielzahl von Filamenten aufweist, und durch eine zentrale Seele gebildet wird, um die herum die Litze ausgebildet ist, wobei die Seele von einem Filamentbündel gebildet wird.

8. Material gemäß Anspruch 1, dadurch gekennzeichnet, daß der Anker (3) aus einem rohrförmigen Körper (3A) gebildet ist, der mit dem flexiblen Verbindungsglied verbunden ist und der in Richtung der Nadel durch zwei gegenüberliegende und auseinanderlaufende Spitzen (3B) verlängert ist, wobei der rohrförmige Körper (3A) auf dem flexiblen Verbindungsglied (2) derart deformiert ist, daß er in seiner Lage auf diesem letzteren blockiert ist.

9. Material gemäß Anspruch 1, dadurch gekennzeichnet, daß die Länge des Verbindungsgliedes (2) zwischen dem Anker (3) und dem hinteren Ende der Nadel gleich mindestens einmal die Länge der Nadel ist.

10. Material gemäß Anspruch 9, dadurch gekennzeichnet, daß die Länge des Verbindungsgliedes (2) zwischen dem Anker (3) und dem hinteren Ende der Nadel ungefähr gleich zwei Nadellängen ist.

11. Material gemäß Anspruch 1, dadurch gekennzeichnet, daß die Scheibe (5) in einer Richtung senkrecht zu der Achse ihrer Öffnung eine ebene erste Fläche (5A) und eine dieser gegenüberliegende konvexe zweite Fläche (5B) aufweist, die zum Andrücken gegen die retromalleoare Grube bestimmt ist.

12. Material gemäß Anspruch 1, dadurch gekennzeichnet, daß das Halteorgan (6) durch ein zylindrisches, axial durchbohrtes Element gebildet ist, das an einem Ende eine konvexe Fläche 6A aufweist, die zum Andrücken gegen die Scheibe (5) bestimmt ist, wobei das Organ aus einem duktilen Material besteht, damit es auf dem Verbindungsglied deformiert werden kann.
